# EUROPEAN PATENT SPECIFICATION

(11) **EP 4 531 673 B1**
(45) Date of publication and mention of the grant of the patent: **03.12.2025**
(21) Application number: 23728332.0
(22) Date of filing: 23.05.2023
(51) Int. Cl.: A61B 5/091, A61B 5/00

(54) **ENDOBRONCHIAL FLOW MEASUREMENT AND FLOW CONTROL FOR REGIONAL VENTILATION**
ENDOBRONCHIALE DURCHFLUSSMESSUNG UND DURCHFLUSSREGELUNG FÜR REGIONALE BEATMUNG
MESURE DE L'ÉCOULEMENT ENDOBRONCHIQUE ET RÉGULATION DE L'ÉCOULEMENT POUR LA VENTILATION RÉGIONALE

(30) Priority: 27.05.2022 US 202263346341 P
(43) Date of publication of application: 09.04.2025
(73) Proprietor: Koninklijke Philips N.V., 5656 AG Eindhoven (NL)
(72) Inventor: SABCZYNSKI, Joerg, 5656 AG Eindhoven (NL); GLEICH, Bernhard, 5656 AG Eindhoven (NL); RAHMER, Juergen Erwin, 5656 AG Eindhoven (NL); KOEHLER, Thomas, 5656 AG Eindhoven (NL); WIEMKER, Rafael, 5656 AG Eindhoven (NL); BUIZZA, Roberto, 5656 AG Eindhoven (NL); HENDRIKS, Cornelis Petrus, 5656 AG Eindhoven (NL); HAARTSEN, Jaap Roger, 5656 AG Eindhoven (NL); WIEBERNEIT, Nataly, 5656 AG Eindhoven (NL)
(74) Representative: Philips Intellectual Property & Standards
(86) International application number: PCT/EP2023/063788
(87) International publication number: WO 2023/227602

(56) References cited:
- EP-A1- 3 299 058
- WO-A1-2010/017685
- WO-A1-2014/144070
- WO-A1-2020/253977
- WO-A1-2021/239484
- CN-A- 113 941 063
- "Pulmonary Diseases - Clinopathological correlations", 1 January 1983, SPRINGER VERLAG BERLIN HEIDELBERG, ISBN: 978-3-642-69136-2, article MÜLLER K.M., XP093069306, DOI: 10.1007/978-3-642-69134-8
- NOMA HAYATO ET AL: "Development of Stent Flow Sensor Device Evaluating Breathing Property at Airway in Experimental Animal Under Free Move Condition", 2020 IEEE 33RD INTERNATIONAL CONFERENCE ON MICRO ELECTRO MECHANICAL SYSTEMS (MEMS), IEEE, 18 January 2020 (2020-01-18), pages 630 - 633, XP033753263, DOI: 10.1109/MEMS46641.2020.9056298

## Description

### CROSS-REFERENCE TO RELATED APPLICATIONS

This patent application claims the priority benefit under 35 U.S.C. § 119(e) of U.S. Provisional Application No. 63/346,341, filed on May 27, 2022.

The following relates generally to the respiratory therapy arts, mechanical ventilation arts, mechanical ventilation control arts, endobronchial valve arts, and related arts.

### BACKGROUND

Invasive mechanical ventilation is a key component of intensive care. Several different methods of ventilation exist, which differ in several variables: Ventilation can be distinguished by the way how a breath is triggered (pressure, volume, flow), by the limits (on volume, pressure, or flow) that should not be exceeded, and the cycle factors terminating an inspiration (again pressure, volume, flow, time). There exist volume-targeted ventilator modes, pressure targeted modes, as well as combined modes. An amount of Positive End-Expiratory Pressure (PEEP) is an important factor as well. The intensivist has thus many ventilation parameters at hand to optimally care for the individual patient.

However, all of these different ventilation modes and parameters provide air, pressure, volume, and flow via a single interface to the patient body: the trachea and therein an endotracheal tube (ETT) for invasive ventilation. The distribution of the insufflated air volume within the lung depends on the local airway resistance and the regional compliance of the respiratory system. In some diseases, one or both of the local airway resistance and the regional compliance are heterogenous, thus leading to heterogeneous distribution of the insufflated volume. This can lead to the situation that in some regions the PEEP value is too low for lung recruitment, while in other regions the pressure is too high and leads to lung overdistention or to cardiac complications. Examples are patients having lobar pneumonia, lobar atelectasis, or other asymmetrical pulmonary problems. Patients with e.g. ARDS (Acute Respiratory Distress Syndrome) often develop an edema, which due to gravity-induced pressure can lead to poorly inflated or collapsed pulmonary units of the dependent lung regions.

Inhomogeneous inflation or deflation of the lungs can also cause dynamic pressure differences between regions and lead to interregional airflows known as pendelluft (see, e.g., Elliot E. Greenblatt et al., 'Pendelluft in the Bronchial Tree,' Journal of Applied Physiology, 117.9 (2014), 979-88 https://doi.org/10.1152/japplphysiol. 00466.2014). This may be an important phenomenon contributing to regional gas exchange, irreversible mixing, and aerosol deposition patterns inside poorly ventilated regions of the lung. It is more prominent in diseases with a significant heterogeneity in both resistance and compliance.

In addition, COPD (Chronic Obstructive Pulmonary Disease) is a progressive lung disease associated with emphysema. Emphysema is characterized by air-filled spaces in the lung and permanent damage to the lung tissue. Emphysematous parts of the lung can hyperinflate. In consequence, other healthier parts of the lung have less space in the thorax and can thus not participate adequately in gas exchange leading to shortness of breath. Therefore, the surgical removal of the lobe most affected by emphysema (lung volume reduction surgery) can be an effective treatment for severe COPD.

The implantation of endobronchial valves is a minimally-invasive alternative to lung volume reduction surgery and is also called Bronchoscopic Lung Volume Reduction (BLVR). One or more endobronchial valves are placed bronchoscopically (i.e. under visual control) in one or more bronchi. These valves are simple one-way valves, which allow the air to be breathed out, but not breathed in. After implantation, the target lobe rapidly collapses (if there is no pulmonary shunt) and gives space to more healthy lobes. Several valve systems are on the market (e.g., from Olympia Spiration, Pulmonx, and so forth). Endobronchial valves can easily be placed and removed again with a dedicated catheter through the working channel of the bronchoscope.

As such, there are many situations in intensive care, in which it would be favorable that a ventilated patient could get regionally targeted ventilation volume. However, it is not possible with current ventilation systems to locally measure the pressure, flow, or volume reaching a subunit of the lung (e.g. a lobe or a segment). It is also not possible to target parts of the lung with a dedicated parameter setting (e.g. pressure, volume, or flow) or with a dedicated pressure, flow, or volume curve.

Documents WO 2010/017685 A1 and CN 113 941 063 A disclose a medical device comprising a sensor for measuring fluid pressure in the trachea and/or bronchi of a patient.

The following discloses certain improvements to overcome these problems and others.

### SUMMARY

The invention is defined by the appended claims 1 to 4.

In one aspect, a medical device for treating an associated patient includes a bronchial sensor device configured to measure fluid pressure in a bronchus of the associated patient; and an electronic controller configured to: receive the fluid pressure data from the bronchial sensor device; and calculate a fluid flow measurement through the bronchus based on the measured fluid pressure.

One advantage resides in providing patients undergoing mechanical ventilation therapy with a regionally targeted ventilation volume.

Another advantage resides in providing a mechanical ventilation system that can locally measure pressure, flow, or volume reaching a subunit of the lung.

Another advantage resides in targeting parts of a lung with a dedicated parameter setting (e.g. pressure, volume, or flow) or with a dedicated pressure, flow, or volume curve for a patient undergoing mechanical ventilation therapy.

Another advantage resides in automatically adjusting settings of a mechanical ventilator to help wean patients off mechanical ventilation therapy.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

FIGURE 1 diagrammatically shows an illustrative mechanical ventilation system in accordance with the present disclosure.
FIGURES 2-5 show example sensor devices used in the system of FIGURE 1.
FIGURES 6A and 6B show an example of an actuator-controlled endobronchial valve.
FIGURE 7 shows an actuator device used in the system of FIGURE 1.

### DETAILED DESCRIPTION

In order to provide a regional measurement of ventilation parameters for a patient undergoing mechanical ventilation therapy, one or more measurement devices are placed endobronchially (i.e. with the help of a bronchoscope) in one or more of the lobar bronchi or segmental bronchi or even smaller bronchi. The measurement device is placed and fixed endobronchially. It contains one or more pressure sensors, preferably based on Micro-Bot technology (see, e.g., J Rahmer, C Stehning, and B Gleich, 'Spatially Selective Remote Magnetic Actuation of Identical Helical Micromachines.,' Science Robotics, 2.3 (2017)), which can be read out wirelessly and do not need a built-in power supply (see, e.g., US 2021/0244305). The measurement device is based on Bernoulli's principle and the configuration of the pressure sensors in the measurement device depends on the physical quantity to be measured, e.g. pressure and flow. Typically, the measurement device has some sort of known constriction, which adds a defined resistor to the airflow. The pressure signal from the pressure sensors is read out and transmitted to a computer, which calculates regional e.g. time-pressure, time-flow, and time-volume curves. Alternatively, regional ventilation parameters can be assessed with the help of imaging devices.

In addition, ventilators can provide ventilation only via the trachea to the lungs in total. Thus, they can ventilate only with a single time-pressure or time-volume curve. To provide individual pressure, volume, or flow to different lung regions (lobes, segments, or even subsegmental regions), one or more control devices are placed endobronchially. These control devices can shape ventilation parameters like pressure, volume, or flow by adapting (typically increasing) the resistance of the airway or acting as a valve and fully closing the airway.

The control device contains one or more actuators, preferably based on Micro-Bot technology, which can be switched wirelessly without built-in power supply (see, e.g., Jürgen Rahmer, Christian Stehning, and Bernhard Gleich, 'Remote Magnetic Actuation Using a Clinical Scale System,' PLOS ONE, 13.3 (2018)). The switching or resistance change is controlled by a computer. This controller may work independently (sensing the ventilator actions), or may be digitally connected to the mechanical ventilator such that the timing and strength of valve actions are in synchrony with the ventilation.

As disclosed herein, in a preferred embodiment, a measurement device and a control device are combined in one device allowing to measure and shape regional ventilation parameters in real-time. The system can work in an open and a closed loop manner and can work continuously or intermittently. Both, measurement and control devices can be built based on Micro-Bot technology comprising magneto-mechanical oscillators, which enable building tiny (1-2 mm in length) wireless markers and sensors that can be interrogated remotely using magnetic fields. Micro-bots can be built such that physical quantities like pressure are influencing their oscillation frequency, which can be detected from the outside. They do not require a power supply and are thus ideal for the application of providing regional ventilation.

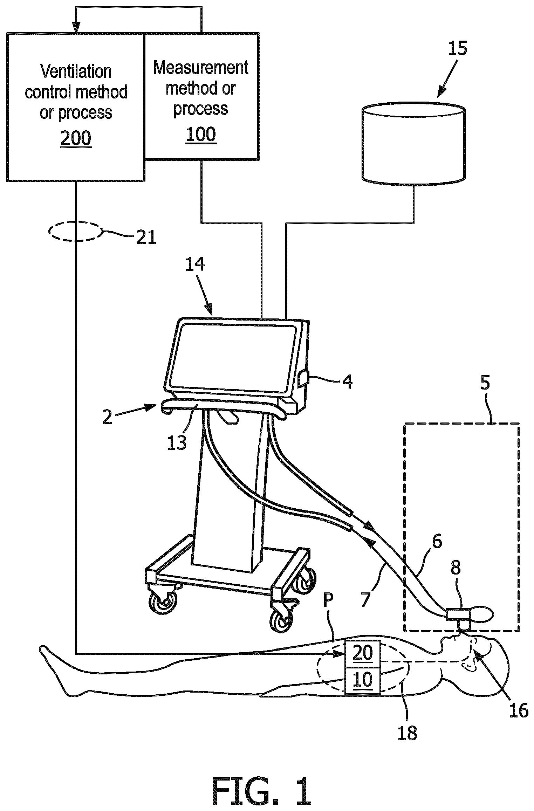

With reference to FIGURE 1, a mechanical ventilator **2** for providing ventilation therapy to an associated patient **P** is shown. As shown in FIGURE 1, the mechanical ventilator **2** includes an outlet **4** connectable with a patient breathing circuit **5** to delivery mechanical ventilation to the patient **P.** The patient breathing circuit **5** includes typical components for a mechanical ventilator, such as an inlet line **6,** an optional outlet line **7** (this may be omitted if the ventilator employs a single-limb patient circuit), a connector or port **8** for connecting with an endotracheal tube (ETT) **16** inserted into the trachea of the patient **P** (or, alternatively, connecting to a tracheostomy tube, not shown), and one or more breathing sensors (not shown), such as a gas flow meter, a pressure sensor, end-tidal carbon dioxide (etCO₂) sensor, and/or so forth. The mechanical ventilator **2** is designed to deliver air, an air-oxygen mixture, or other breathable gas (supply not shown) to the outlet **4** at a programmed pressure and/or flow rate to ventilate the patient via an ETT. The mechanical ventilator **2** also includes an electronic controller **13** (e.g., an electronic processor or a microprocessor), a display device **14,** and a non-transitory computer readable medium **15** storing instructions executable by the electronic controller **13.** The instructions can include fluid (e.g., liquid or gas) flow measurement method or process **100.**

FIGURE 1 diagrammatically illustrates the patient **P** intubated with an ETT **16** (the lower portion of which is inside the patient **P** and hence is shown in phantom). The connector or port **8** connects with the ETT **16** to operatively connect the mechanical ventilator **2** to deliver breathable air to the patient **P** via the ETT **16.** The mechanical ventilation provided by the mechanical ventilator **2** via the ETT **16** may be therapeutic for a wide range of conditions, such as various types of pulmonary conditions like emphysema or pneumonia, viral or bacterial infections impacting respiration such as a COVID-19 infection or severe influenza, cardiovascular conditions in which the patient **P** receives breathable gas enriched with oxygen, or so forth.

FIGURE 1 also shows an endobronchial sensor **10** (diagrammatically shown in FIGURE 1 as a rectangle) disposed in a bronchus (not shown) of diagrammatically indicated lungs **18** of the patient **P.** The sensor **10** comprises a bronchial sensor device **10** configured to measure fluid pressure in the bronchus of the patient **P.** Embodiments of the bronchial sensor device **10** are described herein. FIGURE 1 also shows a magneto-mechanical endobronchial valve **20** (also diagrammatically shown in FIGURE 1 as a rectangle) disposed in the same or a different bronchus of the lungs **18** of the patient **P.** Embodiments of the endobronchial valve **20** are also described herein.

The non-transitory computer readable medium **15** of the mechanical ventilator **2** stores instructions executable by the electronic controller **13** of the mechanical ventilator **2** perform a measurement method or process **100.** To do so, the measurement method **100** performed by the electronic controller **13** includes receiving the fluid pressure data from the bronchial sensor device **10,** and calculating a fluid flow measurement through the bronchus based on the measured fluid pressure. In one embodiment, an indication of the fluid flow measurement can be displayed on the display device **14** of the mechanical ventilator **2.** In another embodiment, the fluid flow measurement can be input to the electronic controller **13** to control operation of the mechanical ventilator in delivery mechanical ventilation therapy to the patient **P.** For example, if the fluid flow measurement underruns a predetermined fluid flow threshold, the mechanical ventilator **2** can be controlled to provide additional air to the patient **P.** whereas if the fluid flow measurement is equal to or exceeds the predetermined fluid flow threshold, the mechanical ventilator **2** can be controlled to provide a lower quantity of air to the patient **P.**

The non-transitory computer readable medium **15** of the mechanical ventilator **2** optionally further stores instructions executable by the electronic controller **13** of the mechanical ventilator **2** perform a ventilation control method or process **100.** To do so, the control method **200** performed by the electronic controller **13** includes the receiving of the fluid flow measurement calculated by the measurement process **100** and, based on that measurement along with other information such as settings of the mechanical ventilator **2,** global ventilation flow measurements acquired by gas flow, pressure, etCO₂, or other sensors of the ventilator **2** generates a control signal **21** for controlling the mechanical ventilation of the patient. In one example, at least one setting of the mechanical ventilator **2** is controlled based on the fluid flow measurement through the bronchus acquired by the method **100.** For example, if the fluid flow measurement indicates that a portion of the lungs **18** may be receiving too much gas flow then the settings of the ventilator **2** may be adjusted to reduce the gas flow measured by the method **100** until it reaches a target level. In another example, if the gas flow measured by the method **100** is too high then the endobronchial valve **20** (which in this example may be in the same bronchus as the sensor **10**) may be actuated to close (completely or partially, depending on the valve type), thereby reducing or eliminating the gas flow through that bronchus. In other examples, at least one setting of the mechanical ventilator **2** can be controlled based on one or more of the measured fluid pressure in the bronchus, the calculated fluid flow measurement through the bronchus, a determined volume of fluid in the bronchus, and other measured or derived parameters.

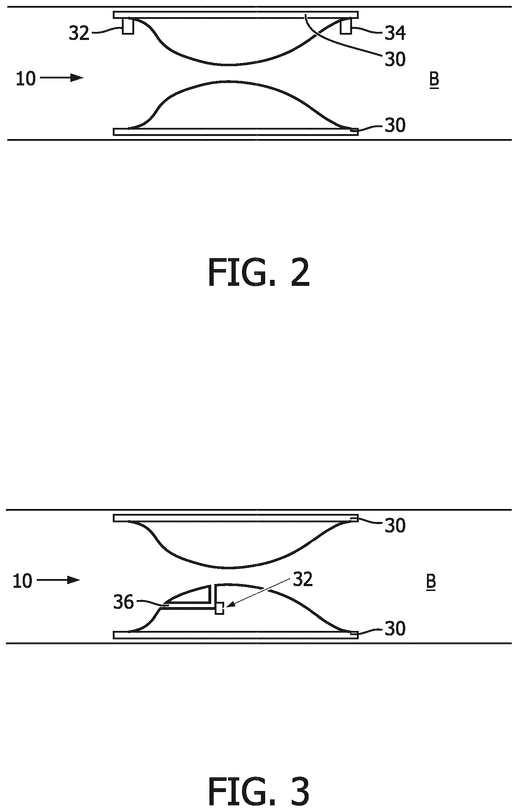

With reference successively to FIGURES 2-5, some nonlimiting illustrative embodiments of the sensor **10** are described.

FIGURE 2 shows one example of the bronchial sensor device **10.** As shown in FIGURE 2, the bronchial sensor device **10** is disposed in the bronchus **B** of the patient **P.** In one example, the bronchial sensor device **10** includes a stent **30** having a diameter smaller than a diameter of the bronchus **B.** A pressure sensor **32, 34** is disposed proximate to each end of the stent **30.** Each pressure sensor **32, 34** comprises a magneto-mechanical oscillator device (i.e., a Micro-Bot). The electronic controller **13** is configured to calculate the fluid flow measurement based on a diameter of the stent **30** and pressure data measured by each pressure sensor **32, 34.** The stent **30** acts a constriction of the airway in the bronchus **B.** According to Bernoulli's law, flow can be calculated from the measured pressure difference over a known resistance, in this case the stent **30** with the known diameter. In another example, the stent **30** has a diameter slightly larger than the diameter of the bronchus **B** such that the stent **30** dilates the bronchus **B** upon insertion therein.

FIGURE 3 shows another example of the bronchial sensor device **10.** As shown in FIGURE 3, the bronchial sensor device **10** is disposed in the bronchus **B** of the patient **P.** The bronchial sensor device **10** includes a tube **36** extending from an end of the stent **30** to a constriction (for air flow) in the stent **30.** A single magneto-mechanical oscillator device **32** is shown (although two or more could be included) and is configured to measure a pressure difference between ends of the constriction in the stent **30.** If the magneto-mechanical oscillator device **32** is placed in the tube **36** from one un-constricted end of the stent **30** to the center of the constriction, the magneto-mechanical oscillator device **32** measures the difference between static pressures between inside and outside of the constriction, which is related to flow. This is an embodiment of the bronchial sensor device **10** as a Venturi meter.

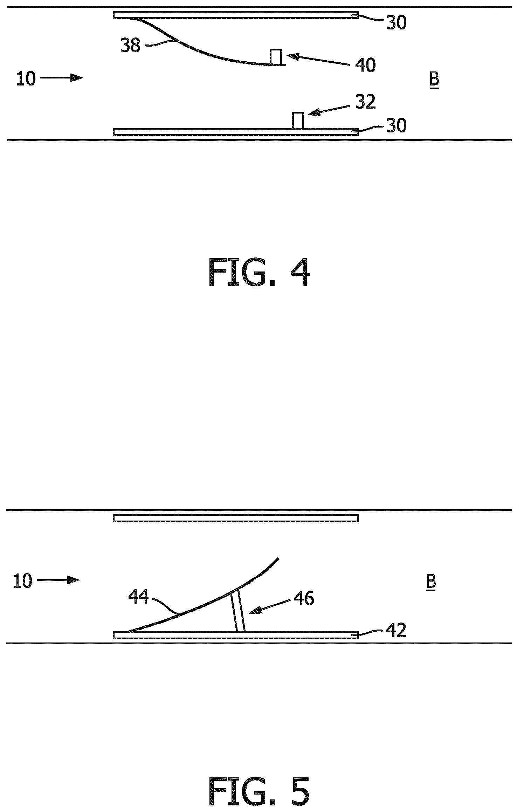

FIGURE 4 shows another example of the bronchial sensor device **10.** As shown in FIGURE 4, the bronchial sensor device **10** is disposed in the bronchus **B** of the patient **P.** The bronchial sensor device **10** includes a deflecting membrane **38** attached to the stent **30** and configured to move based on fluid flow changes in the bronchus **B.** A magnet (i.e., a permanent magnet **40**) is disposed on the deflecting membrane **38.** Each magneto-mechanical oscillator device **32** is configured to measure a pressure difference based on a measured distance between each magneto-mechanical oscillator device **32** and the magnet **40** during movement of the deflecting membrane **38** related to fluid flow in the bronchus **B** (see, e.g., US 2020/0400509).

Yet another method is a configuration similar to a pitot tube where one micro-bot pressure sensor is used to measure the stagnation (or total) pressure and another micro-bot pressure sensor measures the static pressure. From Bernouill's equation the flow velocity-dependent dynamic pressure, and hence the flow velocity, can be calculated.

FIGURES 2-4 depict the bronchial sensor device **10** as a flow measurement device or a volume measurement device. For mechanical ventilation, it is of interest to measure the volume provided to the patient **B** or a region of the lung. If the time-flow curve is measured continuously as it is possible with the help of a flow measurement device, the flow can be integrated over time to get the volume which has been applied.

FIGURE 5 shows an embodiment of the bronchial sensor device **10** configured to control or change the fluid flow. As shown in FIGURE 5, the bronchial sensor device **10** includes a base **42,** a membrane **44** with a hinged connection to the base **42,** and an actuator **46** connected to the membrane **44.** The membrane **44** and the actuator **46** can operate to create a variable constriction, and thus a variable sensor. The electronic controller **13** is configured to calculate the fluid flow based on a distance between the membrane **44** and the base **42** measured by the actuator **46.** In some examples, the actuator **46** comprises a magnet (i.e., a rotating magnet actuated by spatially rotating fields on a simple gear mechanism to adjust an opening size of the constriction), and the electronic controller **13** is configured to calculate the fluid flow based on magnetic fields generated by the magnet **46.** The actuator **46** can also be controlled by, for example, the measured fluid pressure in the bronchus, the calculated fluid flow measurement through the bronchus, a determined volume of fluid in the bronchus, a ventilation parameter of a mechanical ventilator (**2**), and other measured or determined parameters.

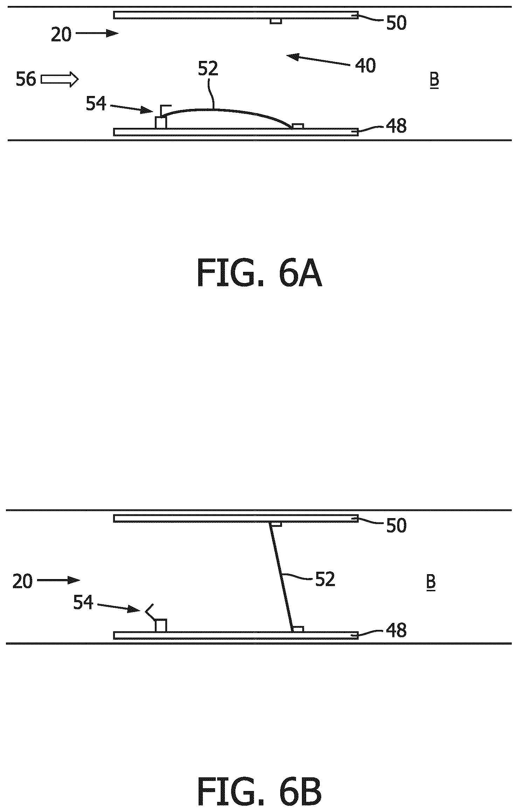

With reference to FIGURES 6A and 6B, some illustrative embodiments of the endobronchial valve **20** are described. In some embodiments, the endobronchial valve **20** can comprise a "active" endobronchial valve by controlling a portion of fluid flowing to the patient **P.**

The endobronchial valve **20** is configured to block a region of the lung, by blocking a bronchial tube that ordinarily passes a portion of the air delivered by the mechanical ventilator **2** to that lung region. As shown in FIGURES 6A and 6B, the endobronchial valve **20** can include a base comprising first and second strips **48, 50** configured to be disposed on opposing walls of the bronchus **B.** A membrane **52** has a pivot connection to the first strip **48.** An actuator **54** (i.e., a magneto-mechanical oscillator device or other magneto-mechanical actuator) is connected to retain the valve in an open position by contacting and retaining the membrane **52** proximate to the second strip **50** as shown in FIGURE 6A, and is operable to release the membrane **52** to close the valve in response to a magnetic control signal to close the valve, as shown in FIGURE 6B. In some examples, the actuator **54** can hold back the membrane **52** in an open position (FIGURE 6A) until the air flow pushes the actuator **54** to the second strip **50** (FIGURE 6B). If this control device is combined with a pressure, flow, or volume sensor, it can be used to limit the maximal pressure, flow, or volume to the lung area of interest. Appropriate safety mechanism can ensure that the valve does not close unintentionally. It is noted that the illustrative valve **20** is a two-position valve, and in the closed position (FIGURE 6B) the air flow may be completely blocked (e.g., if the released membrane **52** completely closes of the lumen) or only partially blocked (e.g., if the membrane **52** has some openings, slits, or the like to allow some passage of air). Moreover, it will be appreciated that the illustrative endobronchial valve **20** of FIGURES 6A and 6B is merely a nonlimiting illustrative example, and other designs are contemplated. For example, there could be two or more (e.g. *N*) membranes **52** presenting different levels of gas flow resistance with a separate actuator for each membrane, and in this way the *N*-membrane valve can generate a range of different discrete flow resistance values.

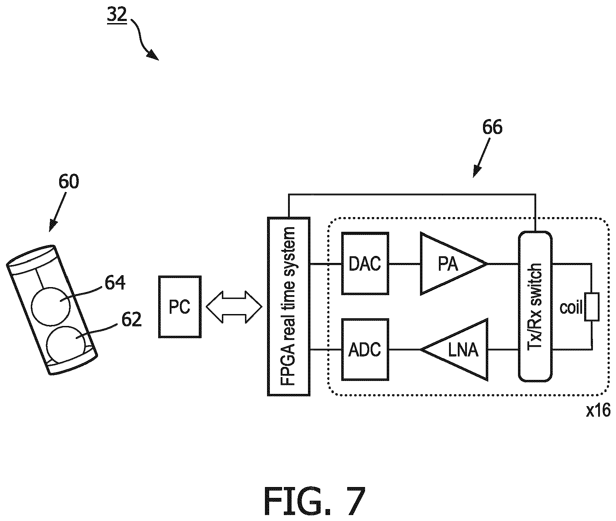

FIGURE 7 shows an example of a magneto-mechanical oscillator device **32.** As shown in FIGURE 7, the magneto-mechanical oscillator device **32** includes a resonator **60** comprising a passive device having a fixed magnet **62** and a suspended permanent magnet **64,** where the suspended permanent magnet **64** performs a rotational oscillation in response to magnetic field pulses from the fixed magnet **62.** The magneto-mechanical oscillator device **32** also includes a detection system **66** configured to generate excitation pulses (transmit channels, Tx) and receiving signal (Rx) from an array of up to 16 coils. The detection system **66** can also apply field sequences for controlling magnetically driven actuators (e.g. rotating fields for screw-based actuators).

In some embodiments, the bronchial sensor device **10** can include a membrane and an adjustable magneto-mechanical oscillator device with a simple gear mechanism that can be used to construct a valve which acts as a pressure regulator. This control device would adjust its opening automatically to limit the pressure to one or more portions of the lungs of the patient **P.** Once the measurement devices combined with control devices have been placed bronchoscopically in the bronchi, these devices are controlled in real-time with the help of a transceiver system to get signals to and from the magneto-mechanical oscillator devices. The signals from the measurement devices are analyzed with the help of a computer program, which calculates pressures, flows, and volumes. These values enter a model of the patient's lung either based on an individual CT scan or on the general knowledge of human anatomy.

The operator of the device **10** can adjust maximal pressure, maximal flow, maximal volume, etc. for each of the control devices in the lung regions of interest. In addition, the computer program controls the settings of a mechanical ventilator, which provides air pressure, flow, and volume through the trachea. Dynamic control of the devices is in particular interesting for handling dynamic ventilation problems like pendelluft by ensuring e.g. that air can flow only out during expiration and only in during inspiration.

As an example use case, alveolar overdistention is a condition which can lead to persistent lung damage after mechanical ventilation. To protect the lungs, a plateau pressure applied to small airways and alveoli during positive-pressure ventilation should be limited. The bronchial sensor device **10** allows for clinicians to measure the local pressure within one or more bronchi **B** of the patient **P.**

Since a local pressure is not necessarily the same as the pressure applied by the mechanical ventilator **2** at the endotracheal tube **16,** local pressure measurements can be used to limit the local plateau pressure, for example, by changing the overall settings of the mechanical ventilator **2** in such a way that the overall plateau pressure at the endotracheal tube **16** is reduced. With this approach, not only the average plateau pressure (as measured by the mechanical ventilator **2**), but also the local plateau pressures, do not exceed a predetermined threshold. For example, the maximum of the local pressure measurements can be used determine the optimal overall pressure by adapting the actual overall pressure (increasing or decreasing) until the maximum of the local pressure measurements has a certain target value.

Instead of measuring the local pressure, it is also possible to measure the local flow and (for example, by integrating flow over time) to measure the local tidal volume using the bronchial sensor device **10.** Therefore, for each of the lung areas equipped with a bronchial sensor device **10** in the corresponding bronchus **B,** it is possible to measure the local tidal volume. If the local tidal volume exceeds a predetermined threshold, the overall tidal volume as applied by the mechanical ventilator **2** may be reduced until all measured local tidal volumes are below their thresholds.

To protect the lungs from local alveolar overdistention, one or more endobronchial valves **20** may be placed endobronchially in different bronchi **B.** These endobronchial valves **20** may contain the micro-bot actuators **54** configured to close a corresponding bronchus **B** triggered by an external signal applied to the micro-bot actuators **54.** In addition, the endobronchial valves **20** may contain one or more micro-bot sensors **32, 34** which measure local pressure and local flow.

The optimal local tidal volumes corresponding to the lung areas controlled by the endobronchial valves **20** may be determined based on a computed tomography (CT) scan or a digital model of a lung of the patient **P.** These optimal local tidal volumes are portions (i.e., percentages) of the overall tidal volume that the mechanical ventilator **2** delivers at the endotracheal tube **16.** If the overall tidal volume is increased or decreased, these optimal local tidal volumes also change proportionally.

If during an inspiration the volume measurement at one of the endobronchial valves **20** exceeds the optimal local tidal volume, the micro-bot actuator **54** is triggered to close the endobronchial valve **20.** Thereafter, a remainder of the overall tidal volume as delivered by the mechanical ventilator **2** would be distributed to the other lung areas, which have not reached their optimal tidal volume yet. The procedure would be repeated with the rest of the endobronchial valves **20** until, at the end of the inspiration (i.e., when the overall tidal volume is delivered by the mechanical ventilator **2**), all lung areas behind the endobronchial valves **20** will have received their optimal tidal volume.

In some embodiments, instead of implanting active control devices, passive resistors or valves can be used. For example, passive resistors are simple stents with constrictions of a defined diameter. They can either be built with fixed diameter, and the resistor with the right diameter must be selected. Alternatively, resistors with adjustable constriction (and thus adjustable resistance) can be built. Their properties are set before implantation and as passive devices remain fixed until they are explanted. In another example, passive valves are similar to passive resistors. They open and close at a fixed (or adjustable and then fixed) pressure or flow.

In some embodiments, a computer program (e.g. a model comprising a Digital Twin of the patient) can be used to estimate the optimal selection or settings of the passive control devices.

In some embodiments, feedback can be provided to a clinician, such as feedback from functional imaging to adapt the control devices and/or feedback from wearables or patient monitors (e.g. vital sign measuring devices) to adapt the control devices.

## Claims

1. A medical device for treating an associated patient undergoing mechanical ventilation therapy, comprising:
a bronchial sensor device (10) configured to measure fluid pressure in a bronchus (B) of the associated patient (P); and
an electronic controller (13) configured to:
receive the fluid pressure data from the bronchial sensor device; and
calculate a fluid flow measurement through the bronchus based on the measured fluid pressure,
**characterized in that** the medical device further comprises:
a bronchial valve (20) including:
a base comprising first and second (48,50) strips configured to be disposed on opposing walls of the bronchus;
a membrane (52) with a pivot connection to the first strip;
a magneto-mechanical actuator (54) connected to the membrane;
wherein the magneto-mechanical actuator is configured to retain the valve in an open position by contacting and retaining the membrane proximate to the second strip, and to close the valve by releasing the membrane in response to a magnetic actuation signal.

2. The medical device of claim 1, wherein the bronchial sensor device comprises a stent (30) with a pressure sensor (32,34) disposed proximate to each end thereof;
wherein the electronic controller is configured calculate the fluid flow measurement based on a diameter of the stent and pressure data measured by each pressure sensor.

3. The medical device of claim 2, wherein each pressure sensor comprises a magneto-mechanical oscillator device.

4. The medical device of claim 1, further comprising:
a mechanical ventilator (2) configured to deliver mechanical ventilation to the associated patient;
wherein at least one setting of the mechanical ventilator is controlled based on at least one of:
the measured fluid pressure in the bronchus;
the calculated fluid flow measurement through the bronchus; and
a determined volume of fluid in the bronchus.

## Patentansprüche

1. Medizinische Vorrichtung zur Behandlung eines zugehörigen Patienten, der sich einer mechanischen Beatmungstherapie unterzieht, umfassend:
eine Bronchialsensorvorrichtung (10), die dazu konfiguriert ist, den Flüssigkeitsdruck in einer Bronchie (B) des zugehörigen Patienten (P) zu messen; und
eine elektronische Steuereinheit (13), die konfiguriert ist zum:
Empfangen der Flüssigkeitsdruckdaten von der Bronchialsensorvorrichtung; und
Berechnen einer Flüssigkeitsflussmessung durch die Bronchien basierend auf dem gemessenen Flüssigkeitsdruck,
**dadurch gekennzeichnet, dass** die medizinische Vorrichtung weiter Folgendes umfasst:
ein Bronchialventil (20), das Folgendes einschließt:
eine Basis, die einen ersten und einen zweiten (48, 50) umfasst, die dazu konfiguriert sind, an gegenüberliegenden Wänden der Bronchie angeordnet zu werden;
eine Membran (52) mit einer Schwenkverbindung zum ersten Streifen;
eine magnetomechanische Betätigungsvorrichtung (54), die mit der Membran verbunden ist;
wobei die magnetomechanische Betätigungsvorrichtung dazu konfiguriert ist, das Ventil in einer offenen Position zu halten, indem sie die Membran in der Nähe des zweiten Streifens berührt und festhält, und das Ventil zu schließen, indem sie die Membran als Reaktion auf ein magnetisches Betätigungssignal freigibt.

2. Medizinische Vorrichtung nach Anspruch 1, wobei die Bronchialsensorvorrichtung einen Stent (30) mit einem Drucksensor (32, 34) umfasst, der in der Nähe jedes Endes davon angeordnet ist;
wobei die elektronische Steuereinheit dazu konfiguriert ist, die Flüssigkeitsdurchflussmessung basierend auf einem Durchmesser des Stents und den von jedem Drucksensor gemessenen Druckdaten zu berechnen.

3. Medizinische Vorrichtung nach Anspruch 2, wobei jeder Drucksensor eine magnetomechanische Oszillatorvorrichtung umfasst.

4. Medizinische Vorrichtung nach Anspruch 1, weiter umfassend
ein mechanisches Beatmungsgerät (2), das zum Zuführen von mechanischer Beatmung an den zugehörigen Patienten konfiguriert ist; und
wobei mindestens eine Einstellung des mechanischen Beatmungsgeräts basierend auf mindestens einem der folgenden Faktoren gesteuert wird:
dem gemessenen Flüssigkeitsdruck in der Bronchie;
der berechneten Flüssigkeitsflussmessung durch die Bronchien und einem bestimmten Flüssigkeitsvolumen in der Bronchie.

## Revendications

1. Dispositif médical pour traiter un patient associé soumis à une thérapie de ventilation mécanique, comprenant :
un dispositif de capteur bronchique (10) configuré pour mesurer une pression de fluide dans une bronche (B) du patient associé (P) ; et
un dispositif de commande électronique (13) configurée pour :
recevoir les données de pression de fluide en provenance du dispositif de capteur bronchique ; et
calculer une mesure de débit de fluide à travers la bronche sur la base de la pression de fluide mesurée,
**caractérisé en ce que** le dispositif médical comprend en outre
une valve bronchique (20) incluant :
une base comprenant une première et une seconde bande (48, 50) configurées pour être disposées sur des parois opposées de la bronche ;
une membrane (52) avec une liaison pivot à la première bande ;
un actionneur magnéto-mécanique (54) relié à la membrane ;
dans lequel l'actionneur magnéto-mécanique est configuré pour maintenir la valve dans une position ouverte en contactant et en retenant la membrane à proximité de la seconde bande, et pour fermer la valve en libérant la membrane à la suite d'un signal d'actionnement magnétique.

2. Dispositif médical selon la revendication 1, dans lequel le dispositif de capteur bronchique comprend une endoprothèse (30) avec un capteur de pression (32, 34) disposé à proximité de chaque extrémité de celui-ci ;
dans lequel le dispositif de commande électronique est configuré pour calculer la mesure de débit de fluide sur la base du diamètre de l'endoprothèse et des données de pression mesurées par chaque capteur de pression.

3. Dispositif médical selon la revendication 2, dans lequel chaque capteur de pression comprend un dispositif d'oscillateur magnéto-mécanique.

4. Dispositif médical selon la revendication 1 comprenant en outre
un ventilateur mécanique (2) configuré pour fournir une ventilation mécanique à un patient ; et
dans lequel au moins un réglage du ventilateur mécanique est commandé sur la base d'au moins un :
de la pression de fluide mesurée dans la bronche ;
de la mesure de débit de fluide calculé à travers la bronche ; et d'un volume déterminé de fluide dans la bronche.
